Europäisches Patentamt

European Patent Office

Office européen des brevets

(11) Veröffentlichungsnummer: **0 237 795 B1**

## EUROPÄISCHE PATENTSCHRIFT

(45) Veröffentlichungstag der Patentschrift: **05.08.92**

(51) Int. Cl.⁵: **A61B 17/39**, H03L 5/02

(21) Anmeldenummer: **87102050.9**

(22) Anmeldetag: **13.02.87**

(54) **Hochfrequenzgenerator mit automatischer Leistungsregelung für die Hochfrequenzchirurgie.**

(30) Priorität: **15.02.86 DE 3604823**

(43) Veröffentlichungstag der Anmeldung:
**23.09.87 Patentblatt 87/39**

(45) Bekanntmachung des Hinweises auf die
Patenterteilung:
**05.08.92 Patentblatt 92/32**

(84) Benannte Vertragsstaaten:
**CH DE ES FR GB IT LI SE**

(56) Entgegenhaltungen:
**EP-A- 0 157 534**
**FR-A- 2 500 708**
**US-A- 3 718 852**
**US-A- 3 921 092**
**US-A- 4 321 483**

(73) Patentinhaber: **MED Inventio AG**
**Seestrasse 359**
**CH-8038 Zürich-Wollishofen(CH)**

(72) Erfinder: **Flachenecker, Gerhard, Prof Dr.-Ing.**
**Bozener Strasse 2**
**W-8012 Ottobrunn(DE)**
Erfinder: **Fastenmeier, Karl, Prof Dr.-Ing.**
**Pasettistrasse 2**
**W-8000 München 83(DE)**
Erfinder: **Lindenmeier, Heinz, Prof Dr.-Ing.**
**Fürstenrieder Strasse 7**
**W-8033 Planegg(DE)**

(74) Vertreter: **Wenzel, Joachim, Dipl.-Ing.**
**Hauptmannsreute 46**
**W-7000 Stuttgart 1(DE)**

## Beschreibung

Die Erfindung betrifft einen Hochfrequenzgenerator mit automatischer Leistungsregelung für die Hochfrequenzchirurgie entsprechend dem Oberbegriff des Anspruchs 1.

Hochfrequenzströme werden in der Chirurgie zum Schneiden von menschlichem Gewebe verwendet, wenn ein besonders blutarmer Schnitt erwünscht ist, oder wenn der Operationsort mit einem normalen Skalpell nicht zugänglich ist, sich aber für ein dünnes Instrument ein Zugang durch natürliche Körperöffnungen wie Speiseröhre, Darm oder Harnröhre anbietet. Beispiele sind die Entfernung von Prostata-Adenomen, Blasentumoren oder Darmpolypen. Mithilfe einer Chirurgiesonde wird dabei der Hochfrequenzstrom in das zu schneidende Gewebe eingespeist. Die entstehende Verlustwärme bringt die Zellflüssigkeit an dieser Stelle zum Sieden und Verdampfen, wobei die Zellwände aufreissen und das Gewebe getrennt wird. Die Frequenz des verwendeten Stromes muß oberhalb ca. 300 kHz liegen, um im Körper des Patienten keine elektrochemischen Reaktionen solcher Größenordnung auszulösen, daß eine Nerven- oder Muskelreizung stattfindet.

Ein Problem der Hochfrequenzchirurgie ist die richtige Leistungsdosierung des Hochfrequenzgenerators. Der Leistungsbedarf beim Schneiden von menschlichem Gewebe hängt von sehr vielen Faktoren ab, wie: Schnittgeschwindigkeit, Abmessungen und Abnutzung der Chirurgiesonde, Beschaffenheit und Durchblutung des zu schneidenden Gewebes, Anwesenheit und Zusammensetzung einer Spülflüssigkeit usw. Diese Faktoren können sich während eines einzigen Schnittes, d.h. innerhalb etwa einer Sekunde, und natürlich erst recht während einer ganzen Operation sehr deutlich ändern. Ein Unterschreiten der momentan notwendigen Leistung resultiert mindestens in einer mangelhaften Schnittqualität oder die Chirurgiesonde erlaubt überhaupt keinen Schnitt mehr. Bei von Hand einstellbaren Hochfrequenzgeneratoren muß der Arzt daher eine Leistungsstufe wählen, die beim höchsten vorkommenden Leistungsbedarf gerade noch ausreichend ist. Dies ist eine Einstellung nach Erfahrung, die in den weitaus größten Zeiten der Operation einen Leistungsüberschuß bedeutet. Dieser Leistungsüberschuß wird in Form eines intensiven Lichtbogens zwischen der Operationssonde und dem zu schneidenden Gewebe aufgebraucht. Hieraus entstehen ernsthafte Nachteile der Hochfrequenzchirurgie. Ein intensiver Lichtbogen erzeugt auf der Schnittfläche eine übermäßige Nekrose, die die Heilung verzögert. Außerdem wirkt ein Lichtbogen zwischen flüssigkeitshaltigem Gewebe und einer metallischen Sonde auf den Hochfrequenzstrom gleichrichtend. Die dabei entstehenden niederfrequenten Ströme können beim Patienten eine Nerven- oder Muskelreizung auslösen, was zu gefährlichen Situationen, wie einem "Springen" des Patienten führen kann.

Aus diesem Grund hat man versucht, die Leistungsabgabe des Hochfrequenzgenerators zu automatisieren und den Hochfrequenzgenerator ständig auf den kleinstmöglichen Wert der Ausgangsleistung zu regeln. Aus der Deutschen Patentschrift 25 04 280 ist eine Vorrichtung zum Schneiden und/oder Koagulieren menschlichen Gewebes mit Hochfrequenzstrom bekannt. Bei dieser Vorrichtung wird die Erkenntnis genutzt, daß zum Gewebeschneiden grundsätzlich ein kleiner Lichtbogen zwischen Chirurgiesonde und Gewebe notwendig ist, um - bei kleinstmöglichem Gesamtstrom - die Stromdichte in dem zu schneidenden Gewebe möglichst groß zu machen. Der Lichtbogen soll so klein wie möglich sein, um die unerwünschten Nebenwirkungen so klein wie möglich, d.h. im allgemeinen vernachlässigbar klein zu halten. In der DP 25 04 280 wird daher vorgeschlagen, die Intensität des Lichtbogens zu messen und mithilfe des Meßsignals auf einen einstellbaren Wert konstant zu regeln. Dazu wird in einer Ausgestaltung die nichtlineare Wirkung des Lichtbogens verwendet. Speist man die Chirurgiesonde mit einer Hochfrequenzspannung einer Frequenz, so erzeugt der Lichtbogen Ströme auf Vielfachen der Generatorfrequenz und im Bereich der Frequenz Null. Man nennt diese neu entstehenden Frequenzen "harmonische Frequenzen" oder die "Harmonischen" der Generatorfrequenz. Die Amplituden dieser zusätzlichen Ströme hängen von der Intensität des Lichtbogens ab. Mißt man die Amplituden der Harmonischen, so hat man ein Maß für die Intensität des Lichtbogens und kann dieses Meßergebnis über eine Leistungsregelung zur Konstanthaltung der Intensität des Lichtbogens verwenden. Regelt man die Intensität des Lichtbogens auf einen sehr kleinen Wert, so hat man gleichzeitig die kleinstmögliche Ausgangsleistung des Hochfrequenzgenerators erreicht.

Dabei ist zu beachten, daß die Amplituden der vom Lichtbogen erzeugten Harmonischen sehr viel kleiner sind als die Amplitude der vom Hochfrequenzgenerator erzeugten Grundschwingung auf der Generatorfrequenz. Weiterhin erzeugt jeder Hochfrequenzgenerator neben der eigentlichen Generatorfrequenz auch selbst harmonische Frequenzen. Dies ist auf die Nichtlinearitäten aller bekannten Verstärkerelemente zurückzuführen. Um die Messung der vom Lichtbogen erzeugten Harmonischen nicht zu stören, müssen die vom Generator erzeugten Harmonischen sehr sorgfältig ausgefiltert werden. In der DP 2504280 wird daher vorgeschlagen, zwischen Hochfrequenzgenerator und Chirurgiesonde ein Ausgangsfilter zu legen, das die Ströme der Generatorfrequenz durchläßt und die Ströme der harmonischen Frequenzen sperrt, wobei die Messung der Harmonischen an dem dem Generator abgewandten Ausgangstor dieses Filters erfolgt. Um weiterhin den Meßvorgang der Harmonischen nicht durch die hohen Spannungen bzw. Ströme auf der Generatorfrequenz zu stören, wird vorgeschlagen, vor das Meßgerät zur Messung der Harmonischen ein Meßfilter zu schalten, das die Generatorfrequenz sperrt und nur die harmonischen Frequenzen durchläßt.

Mit diesen Vorschriften entsteht ein kompliziertes System von Filtern, dessen Dimensionierung sehr schwierig ist, weil weitere Eigenarten des Stromkreises berücksichtigt werden müssen.

Bei der Hochfrequenzchirurgie darf der Arzt nicht durch den Hochfrequenzgenerator und elektrische Zuleitungen zur Chirurgiesonde in seiner Bewegungsfreiheit eingeschränkt werden. Die Leitung vom Hochfrequenzgenerator zur Chirurgiesonde ist deswegen meistens sehr lang und außerdem lose und kompliziert geführt. Auch die Rückleitung von der sog. Neutralen Elektrode zum Generator ist meistens sehr lang. Dadurch entstehen Streuinduktivitäten und Streukapazitäten, die von Operation zu Operation, aber auch während einer Operation schwanken. Diese Blindelemente können die in der DP 25 04 280 beschriebenen Filterkreise verstimmen.

Während der Operation ändert sich weiterhin ständig die Impedanz, die der Patient dem Hochfrequenzgenerator bietet. Durch umfangreiche Messungen haben die Erfinder festgestellt, daß z. B. bei transurethralen Prostata- und Blasenoperationen mit Hochfrequenzströmen Impedanzen zwischen ca. 50 und 5000 Ohm auftreten, mit einer Häufung in dem sehr breiten Bereich zwischen 200 und 1000 Ohm. Bei derart stark schwankenden Patientenimpedanzen ist eine Filterdimensionierung nur noch unter Kompromissen möglich.

Die bei der DP 25 04 280 geforderte hohe Sperrdämpfung für die Harmonischen im Ausgangsfilter bei gleichzeitig geringer Dämpfung der Generatorfrequenz ist in der Praxis nur mit einem Dämpfungspol bei der am stärksten vertretenen dritten Harmonischen erzielbar. Ebenso ist die hohe geforderte Sperrdämpfung des Meßfilters bei der Generatorfrequenz nur mit einem Dämpfungspol erreichbar. Das bedeutet, daß der Hochfrequenzgenerator mit einer festen Generatorfrequenz arbeiten muß, auf die die Filter abgestimmt sind.

Streuinduktivitäten und Streukapazitäten können beim Entwurf des Hochfrequenzgenerators nicht oder nur mit einem Mittelwert berücksichtigt werden und jede Änderung dieser parasitären Elemente im Betrieb führt zu einer Fehlanpassung der Blindkomponente der Patientenimpedanz. Dies hat Einschränkungen in der verfügbaren Generatorleistung zur Folge und natürlich wieder Verstimmungen der Filter. Außerdem arbeitet der Hochfrequenzgenerator bei einer Verstimmung auf eine komplexe Impedanz. Dies hat bei allen bekannten Verstärkerschaltungen Probleme mit der Verlustleistung in den Verstärkerelementen und speziell bei Schaltverstärkern - die man zur Erzielung eines hohen Wirkungsgrades verwendet - Überschwinger in Strom und Spannung beim Schalten zur Folge. Dies kann das Betriebsverhalten des Hochfrequenzgenerators soweit stören, daß er unzuverlässig wird und möglicherweise durch Zerstörung eines Verstärkerelementes ausfällt. Um diese Schwierigkeiten zu mindern, müssen die beschriebenen Filter vielkreisig aufgebaut sein, was sowohl zu einer Erhöhung der Durchgangsdämpfung, d.h. zu einer Reduzierung des Wirkungsgrades, als auch zu einer Verminderung der minimalen Sperrdämpfung, d.h. zur Verschlechterung der Detektion des Lichtbogens, führt.

Der Erfindung liegt daher die Aufgabe zugrunde, einen Hochfrequenzgenerator mit automatischer Leistungsregelung für die Hochfrequenzchirurgie zu schaffen, bei der das Ausgangsfilter möglichst einfach aufgebaut ist, das Meßfilter entweder vollkommen vermieden oder ebenfalls möglichst einfach aufgebaut ist, beide Filter unkritisch bezüglich Schwankungen der Generatorfrequenz sind und Schwankungen des Blindanteils der Patientenimpedanz bzw. der parasitären Blindelemente in den Zuleitungen zum Patienten keinen Einfluß auf die verfügbare Leistung und das Betriebsverhalten des Hochfrequenzgenerators haben.

Diese Aufgabe wird erfindungsgemäß mit den in den Kennzeichen der Ansprüche beschriebenen Mitteln gelöst. Als Oszillator wird eine Schaltung verwendet, deren Schwingfrequenz mit elektronischen Mitteln verstellbar ist. Es wird also bewußt von einer festen Frequenz wie beim Stand der Technik abgewichen. Am Ausgang des Leistungsverstärkers ist eine Phasenmeßeinrichtung eingeschaltet, die die Phasenverschiebung zwischen Ausgangsspannung und Ausgangsstrom des Leistungsverstärkers mißt. Das Ausgangssignal der Phasenmeßeinrichtung wird über einen ersten Regelverstärker so an den Frequenzregeleingang des Oszillators zurückgeführt, daß sich die Oszillatorfrequenz solange ändert, bis die Phasenverschiebung zwischen Ausgangsspannung und Ausgangsstrom des Leistungsverstärkers einen bestimmten, vorgegebenen Wert annimmt. Dieser Wert für die Phasenverschiebung zwischen Ausgangsspannung und Ausgangsstrom entspricht dem Optimalwert, bei dem der Leistungsverstärker optimale Betriebsbedingungen für Leistungsabgabe, Stabilität und eventuell Verzerrungsarmut vorfindet. In der Regel wird dieser Wert für die Phasenverschiebung null sein. Der Leistungsverstärker arbeitet dann auf eine reelle Last.

Mit dieser Frequenznachregelung wird erreicht, daß Änderungen der äußeren parasitären Elemente, wie Leitungsinduktivitäten und Leitungskapazitäten, aber auch Toleranzen der frequenzbestimmenden Glieder im Ausgangsfilter und Veränderungen der Bauteilewerte durch Alterung keinen Einfluß auf das Verhalten des Leistungsverstärkers haben. Weil der Leistungsverstärker immer auf die Impedanz mit der optimalen Phasenlage zwischen Ausgangsspannung und Ausgangsstrom arbeitet, sind Leistungsabgabe und Stabilität optimal. Gleichzeitig sind Störeffekte, wie z.B Spannungsüberschwinger und Schalten unter Stromfluß bei Schaltverstärkern minimiert, wodurch die Erfindung noch den weiteren Vorteil einer wesentlich gesteigerten Betriebssicherheit aufweist.

Die variable Generatorfrequenz macht gegenüber dem Stand der Technik auch im Zweig zur Messung der Lichtbogenintensität, hier also zur Messung der Größe der vom Lichtbogen erzeugten harmonischen Schwingungen, erfinderische Maßnahmen notwendig. Wie bei der Würdigung des Standes der Technik beschrieben, wird das Regelsignal bisher durch Gleichrichtung einer oder mehrerer harmonischen Schwingungen erzeugt. Da die Amplituden der harmonischen Schwingungen sehr viel kleiner sind als die Amplitude der vom Leistungsverstärker erzeugten Grundschwingung, wird nach dem Stand der Technik vor die Harmonischen-Meßeinrichtung ein Filter zur Unterdrückung der Generatorfrequenz geschaltet. Die geforderte hohe Sperrdämpfung kann dabei nur mit einem Dämpfungspol bei der Generatorfrequenz erreicht werden. Diese Lösung ist jedoch bei einer variablen Oszillatorfrequenz nicht mehr anwendbar. Bei einem Hochfrequenzgenerator nach der Erfindung wird daher als Harmonischen-Meßeinrichtung ein phasengesteuerter Gleichrichter verwendet, dessen Steuersignal aus der momentanen Frequenz des Oszillators abgeleitet ist und der nur eine der am Ausgang des Hochfrequenzgenerators enthaltenen Harmonischen gleichrichtet. Das dabei entstehende Ausgangssignal wird dem Modulator über einen zweiten Regelverstärker so zugeführt, daß die sich dabei einstellende momentane Ausgangsleistung am Operationsort einen Lichtbogen genau vorgegebener Intensität erzeugt. Der phasengesteuerte Gleichrichter übernimmt dabei gleichzeitig die Rolle eines adaptiven Filters, das alle nicht gleichzurichtenden Anteile des Spektrums, also auch die Generatorfrequenz, in hohem Maße unterdrückt.

In einer vorteilhaften Ausgestaltung der Erfindung wird als Ausgangsfilter ein Bandpaß verwendet. Dieser Bandpaß ist so dimensioniert, daß alle in der Praxis vorkommenden Generatorfrequenzen innerhalb seiner Grenzfrequenzen liegen, die gleichzurichtende höhere Harmonische aber oberhalb der oberen Grenzfrequenz. Diese Vorschrift soll mit einem Beispiel erläutert werden. Ein von den Erfindern realisierter Hochfrequenzgenerator hat eine nominelle Generatorfrequenz von 400 kHz. In sehr vielen Versuchen wurde festgestellt, daß die Generatorfrequenz von der Phasenmeßeinrichtung im praktischen Betrieb in einem Frequenzbereich von 390 kHz bis 410 kHz hin- und hergeregelt wird. Das Ausgangsfilter wurde daher mit einem Durchlaßbereich von 380 kHz bis 420 kHz versehen, um auch für den Fall der Bauteilealterung noch genügend Reserven vorzusehen. Als gleichzurichtende Harmonische wurde die dritte Harmonische ausgewählt, weil sie die größte Amplitude aufweist und daher das Signal mit dem größten Störabstand ergibt. Die niedrigste Frequenz der dritten Harmonischen liegt demnach bei 1140 kHz. Bei dieser Frequenz hat das realisierte Ausgangsfilter bereits eine Dämpfung von 52dB, was zur Unterdrückung der im Generator erzeugten harmonischen Frequenzen vollkommen ausreichend ist.

Als Ausgangsfilter kann auch ein Tiefpaß verwendet werden. In diesem Fall ist die Grenzfrequenz des Tiefpasses so zu wählen, daß die höchste vorkommende Generatorfrequenz unterhalb, die gleichzurichtende höhere Harmonische jedoch oberhalb dieser Grenzfrequenz liegt.

Der Lichtbogen zwischen Chirurgiesonde und Gewebe wirkt wie eine Stromquelle zur Erzeugung von Strömen harmonischer Frequenzen. Das Meßsignal zur Bestimmung der Intensität des Lichtbogens kann daher direkt aus dem Strom im Patientenstromkreis oder nach einer Umwandlung über eine Spannungsmessung gewonnen werden. In einer Ausgestaltung der Erfindung wird die Ausgangsimpedanz des Ausgangsfilters so gestaltet, daß sie bei der gleichzurichtenden harmonischen Frequenz hochohmig ist. Der vom Lichtbogen erzeugte Strom der betreffenden harmonischen Frequenz erzeugt dann an der Ausgangsimpedanz des Hochfrequenzgenerators eine entsprechende Spannung dieser Frequenz, die der Harmonischen-Meßeinrichtung direkt oder über Koppelglieder zugeführt wird. Solche Koppelglieder können z.B. Koppelkondensatoren, Transformatoren, ohm'sche oder kapazitive Spannungsteiler oder Phasenschieber zur Phasenkorrektur des Meßsignals sein.

In einer weiteren Ausgestaltung der Erfindung wird der vom Lichtbogen erzeugte Strom der harmonischen Frequenz selbst zur Bestimmung der Lichtbogenintensität verwendet. Dazu wird das Ausgangsfilter so gestaltet, daß es an seinen Ausgangsklemmen bei der gleichzurichtenden Harmonischen niederohmig ist. In diesem Fall kann der vom Lichtbogen erzeugte Strom der harmonischen Frequenz im Patientenstromkreis und über die Ausgangsklemmen des Hochfrequenzgenerators ungehindert fließen. Mit Hilfe eines Koppelgliedes wird aus diesem Stromkreis ein Meßsignal ausgekoppelt und der Harmonischen-Meßeinrichtung zugeführt. Hier kann als Koppelglied z.B. ein niederohmiger Widerstand oder ein als Stromwandler geschalteter Übertrager verwendet werden.

Grundsätzlich wirkt ein phasengesteuerter Gleichrichter wie ein Bandpaßfilter mit sehr geringer Bandbreite. Die Bandbreite kann mit einem dem phasengesteuerten Gleichrichter nachgeschalteten Tiefpaß beliebig klein gehalten werden. Aus diesem Grund ist bei geeigneter Dimensionierung dieses Tiefpasses kein Meßfilter vor dem phasengesteuerten Gleichrichter notwendig, da für alle fern liegenden Störfrequenzen, insbesondere also die Generatorfrequenz, jede beliebige Dämpfung eingestellt werden kann. Es sind jedoch Schaltungen bekannt bzw. integrierte Schaltungen verfügbar, die bei kleinen Störpegeln hervorragend arbeiten, aber versagen, wenn die Störamplituden am Eingang der Schaltung gewisse Werte überschreiten. Die Ausgangsspannung eines Hochfrequenzgenerators für die Hochfrequenzchirurgie kann bis zu 1000 V, der Ausgangsstrom bis zu 2 A betragen. Dies sind ganz extreme Störsignale für den phasengesteuerten Gleichrichter, die nur mit aufwendigen, extrem linearen und hochaussteuerbaren Spezialschaltungen beherrscht werden können. Solche Schaltungen sind dem Fachmann zwar bekannt, erfordern aber einen hohen Schaltungsaufwand. In einer weiteren Ausgestaltung der Erfindung wird daher vor den Eingang der Harmonischen-Meßeinrichtung ein Meßfilter zur Unterdrückung der Generatorfrequenz geschaltet. Dieses Meßfilter muß keine sehr hohe Dämpfung für die Generatorfrequenz aufweisen, da es nur den Dynamikbereich der Störung einschränken soll. Insbesondere braucht es keinen Dämpfungspol bei der Generatorfrequenz aufzuweisen und schränkt daher die Anwendung einer variablen Generatorfrequenz nicht ein. Das Meßfilter ist daher sehr viel einfacher zu dimensionieren als nach dem Stand der Technik.

In einer Ausgestaltung der Erfindung wird als Meßfilter ein Bandpaß verwendet, dessen Grenzfrequenzen alle Frequenzen einschließen, die die gleichzurichtende Harmonische annehmen kann. Mit den Frequenzen des oben angegebenen Beispiels wäre, gleiche Reserven bei der Dimensionierung vorausgesetzt, ein Durchlaßbereich von 1140kHz bis 1260kHz zu wählen. Bereits ein Filter ersten Grades hätte dann bei der Generatorfrequenz eine Dämpfung von ca. 26dB, was als Vordämpfung der Störung für den phasengesteuerten Gleichrichter im allgemeinen ausreicht.

Wegen der Filtereigenschaft des phasengesteuerten Gleichrichters muß das Meßfilter nicht unbedingt die übrigen, nicht zur Gleichrichtung vorgesehenen Harmonischen ausfiltern. In einer weiteren Ausgestaltung der Erfindung wird daher als Meßfilter ein Hochpaß verwendet, dessen Grenzfrequenz zwischen der höchsten vorkommenden Grundschwingung und der niedrigsten vorkommenden Harmonischen liegt, die zur Gleichrichtung vorgesehen ist. Dieses Meßfilter dient damit wieder lediglich als Übersteuerungsschutz gegen eine unzulässige Aussteuerung des phasengesteuerten Gleichrichters durch die Generatorfrequenz.

Phasengesteuerte Gleichrichter bewerten die Phasenverschiebung zwischen der Steuerspannung und der zu messenden Spannung. Sie geben bei einer ganz bestimmten Phasenverschiebung eine maximale Spannung ab, die der Amplitude der gleichzurichtenden Spannung entspricht. Bei den meisten Schaltungen von phasengesteuerten Gleichrichtern ist diese Phasenverschiebung null Grad. Es sind aber auch Schaltungen bekannt, bei denen die notwendige Phasenverschiebung 90 Grad beträgt. Weicht die Phasenlage zwischen Steuerspannung und zu messender Spannung von dieser Phasenlage ab, so gibt der phasengesteuerte Gleichrichter eine kleinere, meist einer cosinus-Funktion folgende Spannung ab. Um wirklich die Amplitude der gleichzurichtenden Harmonischen zu messen, müssen die Steuerspannung und die Meßspannung diese richtige Phasenlage zueinander besitzen. Nun kann die gleichzurichtende Harmonische längs des Meßzweiges Phasenverschiebungen erleiden. Wird z.B. die gleichzurichtende Harmonische aus der Ausgangsspannung des Hochfrequenzgenerators abgeleitet, so wird diese an der Ausgangsimpedanz des Ausgangsfilters gebildet. Weil dieses Filter bei der Frequenz der gleichzurichtenden Harmonischen in der Regel eine imaginäre Ausgangsimpedanz besitzt, entsteht hier eine Phasenverschiebung von 90 Grad. Eine weitere Phasenverschiebung kann in einem eventuell verwendeten Meßfilter entstehen. In einer Ausgestaltung der Erfindung wird daher ein Phasenschieber vor den Steuereingang des phasengesteuerten Gleichrichters geschaltet, mit dem die optimale Phasenverschiebung zwischen der Steuerspannung und der Meßspannung eingestellt werden kann. In einer anderen Ausgestaltung der Erfindung ist ein Phasenschieber vor den Meßeingang des phasengesteuerten Gleichrichters geschaltet, mit dem ebenfalls die optimale Phasenverschiebung zwischen Steuerspannung und Meßspannung eingestellt werden kann.

In einer weiteren Ausgestaltung der Erfindung wird als phasengesteuerter Gleichrichter ein Quadraturdemodulator verwendet. Dieser Demodulator kann z.B. nach an sich bekannten Regeln aus zwei phasengesteuerten Gleichrichtern aufgebaut sein, die mit zwei orthogonalen, also um 90 Grad phasenverschobenen Steuerspannungen gesteuert werden. Als Ergebnis liefert der Quadraturdemodulator zwei Signale A1 und A2, die mithilfe der Beziehung

$$A = \sqrt{A1^2 + A2^2}$$

oder nach anderen, diese Beziehung hinreichend genau nachbildenden Gesetzen zur wirklichen Amplitude A der gleichzurichtenden Harmonischen zusammengesetzt werden. Schaltungen, die eine solche Signalkombination vornehmen, sind dem Fachmann bekannt. Das Signal A, das nun keine Abhängigkeit von der Phasenlage der zu messenden Harmonischen besitzt, wird dem zweiten Regelverstärker zugeführt und zur Regelung der Ausgangsleistung des Hochfrequenzgenerators benutzt. Dem Fachmann sind noch weitere Schaltungen bekannt, die unabhängig von der Phasenlage der Meßspannung bezogen auf die Steuerspannung ein der Amplitude der gleichzurichtenden Harmonischen proportionales Ausgangssignal liefern. Diese Schaltungen können ebenfalls in einem Hochfrequenzgenerator nach der Erfindung Verwendung finden.

Hochfrequenzgeneratoren werden in der Hochfrequenzchirurgie immer nur mit kurzer Einschaltdauer betrieben. Ein Schnitt dauert in der Regel zwischen etwa einer und fünf Sekunden. Zur Vorbereitung des nächsten Schnittes vergehen dann mindestens einige Sekunden. Damit die Regelschleife zur Einstellung der Generatorfrequenz nicht bei jedem Schnittbeginn die optimale Generatorfrequenz von beliebigen Startbedingungen aus einstellen muß, wird in einer weiteren Ausgestaltung der Erfindung zwischen die Phasenmeßeinrichtung und den ersten Regelverstärker ein Sample-and-Hold-Verstärker eingeschaltet. Dieser Sample-and-Hold-Verstärker wird so geschaltet, daß er nur dann das aktuelle Ausgangssignal der Phasenmeßeinrichtung an den ersten Regelverstärker weitergibt, wenn der Generator gerade vom Arzt aktiviert ist. Sobald der Generator vom Arzt abgeschaltet wird, ist der Sample-and-Hold-Verstärker in den Hold-Status gesteuert und gibt bis zum Beginn der nächsten Generatoraktivierung ständig das letzte, während einer Aktivierungsphase des Generators von der Phasenmeßeinrichtung abgegebene Signal an den ersten Regelverstärker weiter. Damit kann der Hochfrequenzgenerator bei jeder Aktivierung mit dem bestmöglichen Schätzwert für die optimale Frequenz starten.

Viele Hochfrequenzgeneratoren für die Hochfrequenzchirurgie haben auch einen Koagulationsmodus zur Stillung von Blutungen, die während des Hochfrequenzschneidens auftreten. Im Koagulationsbetrieb wird die Hochfrequenzleistung nicht kontinuierlich an den Patienten abgegeben, sondern in kurzen Hochfrequenzimpulsen. Diese Hochfrequenzimpulse können sehr kurz, also z.B. nur wenige Hochfrequenzperioden lang sein. Die Zeitdauer eines solchen Hochfrequenzimpulses kann insbesondere so kurz sein, daß der Frequenzregelkreis in dieser Zeit nicht einschwingen kann. In einer weiteren Ausgestaltung der Erfindung wird daher der Sample-and-Hold-Verstärker auch während der Generatoraktivierungen im Koagulationsmodus im Hold-Status belassen und an den ersten Regelverstärker wird dauernd das letzte während einer Schneid-Aktivierung des Hochfrequenzgenerators von der Phasenmeßeinrichtung abgegebene Signal weitergegeben. Damit arbeitet der Hochfrequenzgenerator während der Koagulationsphasen mit einer Frequenz, die während des Schneidens von der Frequenzregelung als optimal ermittelt wurde.

Der Lichtbogen zwischen der Chirurgiesonde und dem zu schneidenden Gewebe ist ein Wechselstromlichtbogen. Aus Symmetriegründen werden von einem solchen Lichtbogen vorwiegend ungeradzahlige Harmonische erzeugt. Erst bei größerer Intensität des Lichtbogens, die durch die Leistungsregelung verhindert werden soll, treten wegen unterschiedlicher Austrittsarbeiten und Temperaturen der Chirurgiesonde und des Gewebes deutlichere Unsymmetrien auf, die auch zu geradzahligen Harmonischen einschließlich der Frequenz Null führen. In jedem Fall nehmen aber die Amplituden der Harmonischen mit steigender Ordnungszahl ab. Aus diesem Grund ist die dritte Harmonische mit der größten Amplitude vorhanden und wird in einer vorteilhaften Ausführung zur Gleichrichtung und damit zur Gewinnung des Signals zur Leistungsregelung herangezogen.

Zur Veranschaulichung der Erfindung sind noch Abbildungen beigefügt.

Es zeigen:

Bild 1:     Blockschaltung eines Hochfrequenzgenerators nach der Erfindung.

Bild 2:     Beispiel einer Spannungsauskopplung der Harmonischen am Ausgang des Ausgangsfilters.

Bild 3:     Beispiel einer Stromauskopplung der Harmonischen am Ausgang des Ausgangsfilters.

Bild 4:     Ausführung eines Quadraturdemodulators zur phasenunabhängigen Bestimmung der Amplitude A einer Harmonischen.

Bild 1 zeigt die Blockschaltung eines Hochfrequenzgenerators nach der Erfindung mit mehreren der in den Unteransprüchen beschriebenen Ausgestaltungen. Der Oszillator 1 speist einen Modulator 2, mit dessen Hilfe die Amplitude der Ausgangsspannung bzw. die Ausgangsleistung geregelt werden kann. Am Ausgang des Leistungsverstärkers 3 liegt die Phasenmeßeinrichtung 4, die die Phasenverschiebung zwischen der Ausgangsspannung und dem Ausgangsstrom des Leistungsverstärkers mißt. Auf die Phasenmeßeinrichtung 4 folgt das Ausgangsfilter 5 und eine Einrichtung 6 zur Auskopplung der Harmonischen. Schließlich folgt die Chirurgiesonde 7.

Die Phasenmeßeinrichtung muß nicht unbedingt, wie in diesem Beispiel angegeben, sowohl Ausgangsspannung als auch Ausgangsstrom unmittelbar hinter dem Leistungsverstärker abgreifen. Wenn z.B. das Ausgangsfilter als Bandpaß aufgebaut ist und das erste Glied aus einem Serienresonanzkreis besteht, so ist der Strom auch noch nach diesem ersten Glied des Filters identisch mit dem Ausgangsstrom des Leistungsverstärkers. In diesem Fall kann das Stromsignal für die Phasenmeßeinrichtung auch aus dem Filter selbst entnommen werden. Damit soll klar gemacht werden, daß die hier beschriebene Reihenfolge der einzelnen Komponenten beispielhaft zu verstehen ist und in der Praxis variieren kann, ohne den Erfindungsgedanken zu verlassen.

Das Ausgangssignal der Phasenmeßeinrichtung 4 wird über den ersten Regelverstärker 8 auf den Oszillator 1 zurückgeführt. Dabei wird das Ausgangssignal der Phasenmeßeinrichtung 4 mit einem Sollwert 9 verglichen, der so gewählt ist, daß er dem optimalen Phasenwert entspricht. Dieser Wert ist in der Regel null.

Das mit den Koppelelementen 6 ausgekoppelte Signal wird der Harmonischen-Meßeinrichtung 10 zugeführt. Die Harmonischen-Meßeinrichtung 10 enthält einen phasengesteuerten Gleichrichter 11 und einen Tiefpaß 12, wobei letzterer zur Einstellung der Meßbandbreite dient. In der Regelungstechnik wird der Tiefpaß 12 auch Schleifenfilter genannt, weil er das Frequenzverhalten der Regelschleife, hier der Leistungsregelschleife bestimmt. Um optimales Leistungsregelverhalten zu erreichen, ist dieses Schleifenfilter 12 nach komplizierten, dem Fachmann jedoch bekannten Regeln zu dimensionieren. Es kann jedoch festgestellt werden, daß das Schleifenfilter hier grundsätzlich Tiefpaßverhalten hat.

Vor den Meßeingang 13 des phasengesteuerten Gleichrichters ist in diesem Ausführungsbeispiel ein Meßfilter 14 geschaltet, in diesem Beispiel ein Hochpaß. Der Hochpaß 14 ist so dimensioniert, daß seine Grenzfrequenz zwischen der höchsten vorkommenden Generatorfrequenz und der niedrigsten vorkommenden gleichzurichtenden harmonischen Frequenz liegt. Er dämpft die im Meßsignal des phasengesteuerten Gleichrichters enthaltene Generatorfrequenz so weit, daß der Linearitätsbereich des phasengesteuerten Gleichrichters nicht überschritten wird. Damit kann als phasengesteuerter Gleichrichter z.B. eine handelsübliche integrierte Schaltung mit kleinem Linearitätsbereich verwendet werden. Bei entsprechender Dimensionierung des phasengesteuerten Gleichrichters mit hohem Linearitätsbereich wäre das Meßfilter 14 nicht nötig.

Dem phasengesteuerten Gleichrichter 11 wird an seinem Steuereingang 15 das Steuersignal zugeführt, das aus der Oszillatorschwingung abgeleitet ist. Das Steuersignal und die gleichzurichtende Harmonische müssen gleiche Frequenz und eine vom Arbeitsprinzip des phasengesteuerten Gleichrichters abhängige, feste Phase zueinander haben. In diesem Ausführungsbeispiel wird die Frequenz der Steuerspannung dadurch gebildet, daß im Oszillator 1 ein Steueroszillator 16 verwendet wird, der auf der n-fachen Generatorfrequenz schwingt, wobei n die Ordnung der gleichzurichtenden Harmonischen ist. Soll z.B. die dritte Harmonische gleichgerichtet werden, so ist n = 3 und der Steueroszillator 16 schwingt auf der dreifachen Generatorfrequenz. Die Steuerspannung für den phasengesteuerten Gleichrichter 11 wird hier direkt vom Steueroszillator 16 abgegriffen, während die Generatorfrequenz durch einen Frequenzteiler 17 mit dem Teilungsverhältnis n:l gebildet wird. Das Ausgangssignal des Frequenzteilers 17 stellt die eigentliche Oszillatorschwingung dar.

Vor den Steuereingang 15 des phasengesteuerten Gleichrichters 11 ist ein Phasenschieber 18 eingeschaltet. Seine Phasenverschiebung bei der n-fachen Generatorfrequenz ist so eingestellt, daß die Steuerspannung am Steuereingang 15 und die gleichzurichtende Harmonische am Meßeingang 13 des phasengesteuerten Gleichrichters 11 genau die gegenseitige Phasenverschiebung aufweisen, bei der der phasengesteuerte Gleichrichter 11 das maximale Ausgangssignal liefert.

Nach Durchlaufen des Schleifenfilters 12 wird das Ausgangssignal 19 des phasengesteuerten Gleichrichters 11 dem zweiten Regelverstärker 20 als Eingangssignal 21 zugeführt. Der Regelverstärker 20 vergleicht das Signal 21 mit einem Sollwert 22, mit dem die momentan gewünschte Intensität des Lichtbogens an der Chirurgiesonde 7 vorgewählt werden kann. Das Ausgangssignal des zweiten Regelverstärkers 20 wird schließlich dem Modulator 2 zugeführt, der die notwendige Ausgangsleistung des Hochfrequenzgenerators einstellt.

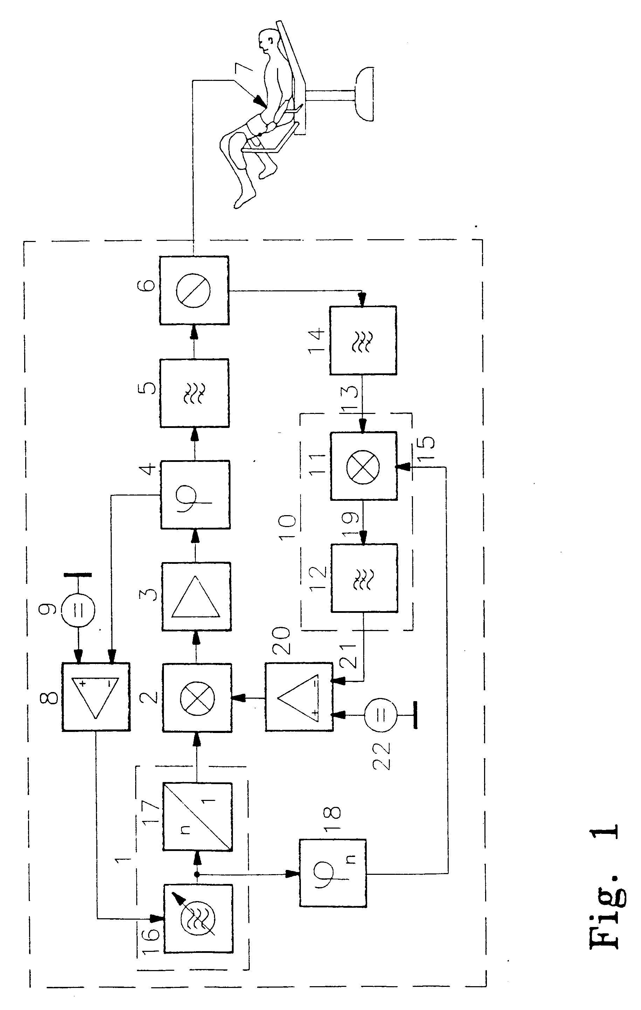

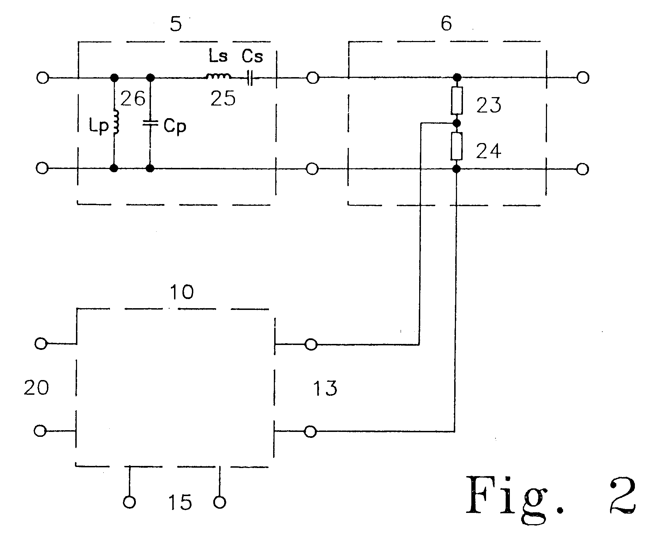

In Fig. 2 ist ein Beispiel für die Ableitung der gleichzurichtenden Harmonischen aus der Ausgangsspannung des Hochfrequenzgenerators dargestellt. Hierzu wird der aus den Widerständen 23 und 24 gebildete Spannungsteiler verwendet. Der Lichtbogen an der Chirurgiesonde 7 wirkt bei der Erzeugung der Harmonischen als Stromquelle. Damit aus dem Strom der gleichzurichtenden Harmonischen eine Spannung entsteht, muß die Ausgangsimpedanz des Ausgangsfilters 5 bei der gleichzurichtenden harmonischen Frequenz hochohmig sein. In dem Ausführungsbeispiel der Fig. 2 ist diese Bedingung mit der angegebenen Schaltung eines Bandpasses für die Generatorfrequenz erfüllt. Der aus Ls und Cs gebildete Serienschwingkreis 25 hat seine Serienresonanz bei der nominellen Generatorfrequenz und ist bei allen harmonischen Frequenzen hochohmig.

Die dargestellte Schaltung ist nur als Beispiel zu verstehen. Der aus den Schwingkreisen 25 und 26 aufgebaute Bandpaß kann nach an sich bekannten Regeln auch in anderen Schaltungsvarianten ausgeführt werden, solange die Ausgangsimpedanz des Ausgangsfilters 5 die Bedingung der Hochohmigkeit bei der gleichzurichtenden Harmonischen erfüllt. Der Bandpaß kann auch Übertrager enthalten, insbesondere wenn der Leistungsverstärker als Gegentaktverstärker aufgebaut ist.

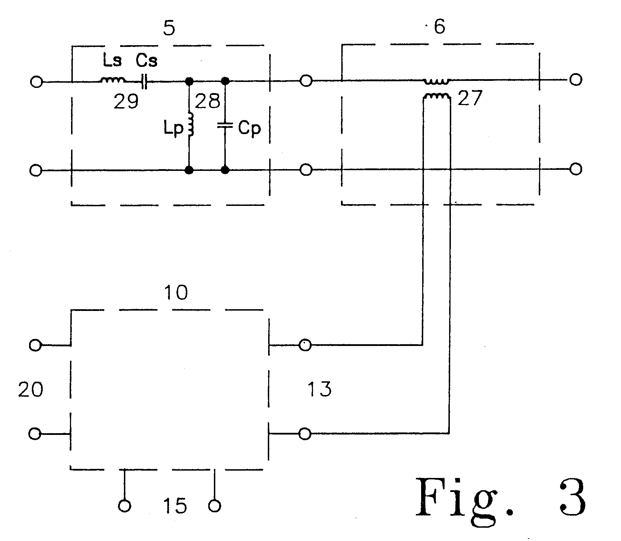

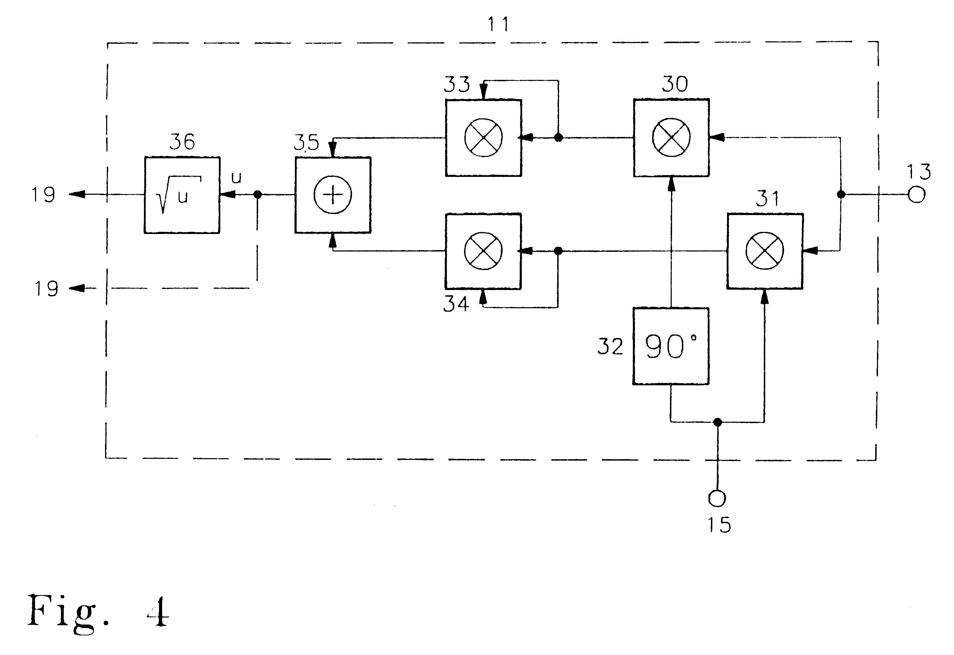

Fig. 3 zeigt eine Schaltung, bei der die gleichzurichtende Harmonische aus dem Ausgangsstrom des Hochfrequenzgenerators abgeleitet wird. Dazu wird ein als Stromwandler geschalteter Übertrager 27 in einer der Ausgangsleitungen des Hochfrequenzgenerators verwendet. In diesem Fall muß der vom Lichtbogen an der Chirurgiesonde 7 erzeugte Strom der gleichzurichtenden harmonischen Frequenz im Ausgangskreis des Hochfrequenzgenerators ungehindert fließen können. Diese Bedingung ist in dem angegebenen Ausführungsbeispiel mit dem Parallelresonanzkreis 28 erfüllt, der Bestandteil des auf die Generatorfrequenz abgestimmten Bandpasses aus den Schwingkreisen 28 und 29 ist. Auch diese Schaltung ist nur beispielhaft zu verstehen.

Das Beispiel eines Blockschaltbildes für einen Quadraturdemodulator ist in Fig. 4 angegeben. Das dem Meßeingang 13 zugeführte Meßsignal, das die gleichzurichtende Harmonische enthält, wird gleichzeitig auf die Meßeingänge der zwei phasengesteuerten Gleichrichter•30 und 31 gegeben. Das am Steuereingang 15 anliegende Steuersignal wird dem Steuereingang des einen phasengesteuerten Gleichrichters 31 unverändert und dem des anderen um 90 Grad phasenverschoben zugeführt. Dadurch entstehen an den Ausgängen der phasengesteuerten Gleichrichter zwei orthogonale Komponenten der gleichzurichtenden Harmonischen. Diese beiden Signalen werden in den Autokorrelatoren 33 und 34 quadriert und im Additionsglied 35 addiert. Das dabei entstehende Ausgangssignal 19 entspricht dem Quadrat der Amplitude der gleichzurichtenden Harmonischen. Bereits dieses Signal kann als Regelsignal weiterverarbeitet werden, wie in Fig. 4 mit der gestrichelten Ausgangsleitung zu sehen ist, weil die Regelaufgabe lediglich lautet, die Amplitude der gleichzurichtenden Harmonischen konstant zu halten. Dabei genügt es natürlich auch, das Quadrat dieser Amplitude konstant zu halten. Als Sollwert 22 muß dann lediglich das Quadrat dieser Amplitude, das ebenfalls ein Maß für die Lichtbogenintensität ist, eingestellt werden.

Ist die sich daraus ergebende Nichtlinearität des Sollwertes 22 unerwünscht, oder ergeben sich Stabilitätsprobleme wegen der Nichtlinearität der Regelschleife, so kann das Signal noch über einen Radizierverstärker 36 nachverarbeitet werden, wie in Fig. 4 angegeben ist.

Der Vorteil eines Quadraturdemodulators ist seine Unabhängigkeit gegenüber Phasenänderungen der Meßspannung. Quadraturdemodulatoren, die nach dem in Fig. 4 beschriebenen Schema wirken oder auch nach anderen Prinzipien arbeiten, sind als integrierte Schaltungen verfügbar. Bei Anwendung einer integrierten Schaltung ist allerdings der Aussteuerbereich zu beachten und im allgemeinen ein Meßfilter zu verwenden, wie im Erfindungsgedanken offenbart ist.

**Patentansprüche**

1. Hochfrequenzgenerator mit automatischer Leistungsregelung für die Hochfrequenzchirurgie, bestehend aus einem Oszillator zur Erzeugung der Generatorfrequenz, einem Modulator zur Regelung der Ausgangsamplitude, einem Leistungsverstärker zur Erzeugung der notwendigen Hochfrequenzleistung, einem Ausgangsfilter zur Unterdrückung von anderen Frequenzen als der Generatorfrequenz des Hochfrequenzgenerators und einer Harmonischen-Meßeinrichtung zur Messung der zur Generatorfrequenz harmonischen Frequenzen im Ausgangskreis des Hochfrequenzgenerators, die von dem zwischen der Chirurgiesonde und dem zu schneidenden Gewebe beim Schneidvorgang existierenden Lichtbogen erzeugt werden, dadurch gekennzeichnet, daß

a) die Frequenz des Oszillators (1) mit elektronischen Mitteln verstellbar ist,

b) am Ausgang des Leistungsverstärkers (3) eine Phasenmeßeinrichtung (4) vorhanden ist, die die Phasenverschiebung zwischen Ausgangsspannung und Ausgangsstrom des Leistungsverstärkers (3) mißt,

c) das Ausgangssignal der Phasenmeßeinrichtung (4) mithilfe eines ersten Regelverstärkers (8) so an den Frequenzregeleingang des Oszillators (1) zurückgeführt ist, daß die Phasenverschiebung zwischen Ausgangsspannung und Ausgangsstrom des Leistungsverstärkers (3) bei der sich dabei einstellenden Frequenz dem Idealwert für den Leistungsverstärker (3), in der Regel also ungefähr null Grad, entspricht und

d) die Harmonischen-Meßeinrichtung (10) einen phasengesteuerten Gleichrichter (11) enthält, dessen Steuersignal aus der momentanen Frequenz des Oszillators (1) abgeleitet ist und der nur eine der im Frequenzgemisch am Ausgang des Hochfrequenzgenerators enthaltene harmonische Frequenz gleichrichtet, und das Ausgangssignal der Harmonischen-Meßeinrichtung (10) dem Modulator (2) über einen zweiten Regelverstärker (20) als Regelsignal zur Regelung der Ausgangsleistung des Hochfrequenzgenerators zugeführt ist.

2. Hochfrequenzgenerator mit automatischer Leistungsregelung für die Hochfrequenzchirurgie nach Anspruch 1, dadurch gekennzeichnet, daß
das Ausgangsfilter (5) ein Bandpaß ist, dessen Grenzfrequenzen so dimensioniert sind, daß alle im Betrieb vorkommenden Generatorfrequenzen innerhalb dieser Grenzfrequenzen liegen, die gleichzurichtende harmonische Frequenz aber außerhalb der Grenzfrequenzen.

3. Hochfrequenzgenerator mit automatischer Leistungsregelung für die Hochfrequenzchirurgie nach Anspruch 1, dadurch gekennzeichnet, daß
das Ausgangsfilter (5) ein Tiefpaß ist, dessen Grenzfrequenz so dimensioniert ist, daß alle im Betrieb vorkommenden Generatorfrequenzen unterhalb dieser Grenzfrequenz liegen, die gleichzurichtende harmonische Frequenz aber oberhalb der Grenzfrequenz.

4. Hochfrequenzgenerator mit automatischer Leistungsregelung für die Hochfrequenzchirurgie nach Anspruch 1 bis 3, dadurch gekennzeichnet, daß
die Ausgangsimpedanz des Ausgangsfilters (5) bei der gleichzurichtenden harmonischen Frequenz hochohmig ist und das der Harmonischen-Meßeinrichtung (10) zugeführte Signal aus der Ausgangsspannung des Hochfrequenzgenerators abgeleitet ist.

5. Hochfrequenzgenerator mit automatischer Leistungsregelung für die Hochfrequenzchirurgie nach Anspruch 1 bis 3, dadurch gekennzeichnet, daß
die Ausgangsimpedanz des Ausgangsfilters (5) bei der gleichzurichtenden harmonischen Frequenz niederohmig ist und das der Harmonischen-Meßeinrichtung (10) zugeführte Signal aus dem Ausgangsstrom des Hochfrequenzgenerators abgeleitet ist.

6. Hochfrequenzgenerator mit automatischer Leistungsregelung für die Hochfrequenzchirurgie nach Anspruch 1 bis 5, dadurch gekennzeichnet, daß
vor die Harmonischen-Meßeinrichtung (10) ein Meßfilter (14) zur Unterdrückung der Generatorfrequenz geschaltet ist, dessen Dämpfung bei allen vorkommenden Generatorfrequenzen so hoch ist, daß der Linearitätsbereich des phasengesteuerten Gleichrichters (11) durch das Meßsignal nicht überschritten wird.

7. Hochfrequenzgenerator mit automatischer Leistungsregelung für die Hochfrequenzchirurgie nach Anspruch 6, dadurch gekennzeichnet, daß
das Meßfilter (14) ein Bandpaß ist, dessen Grenzfrequenzen so dimensioniert sind, daß die gleichzurichtende harmonische Frequenz bei allen im Betrieb vorkommenden Generatorfrequenzen innerhalb dieser Grenzfrequenzen liegt, die Generatorfrequenz jedoch außerhalb der Grenzfrequenzen.

8. Hochfrequenzgenerator mit automatischer Leistungsregelung für die Hochfrequenzchirurgie nach Anspruch 6, dadurch gekennzeichnet, daß
das Meßfilter (14) ein Hochpaß ist, dessen Grenzfrequenz so dimensioniert ist, daß die gleichzurichtende harmonische Frequenz bei allen im Betrieb vorkommenden Generatorfrequenzen oberhalb dieser Grenzfrequenz liegt, die Generatorfrequenz jedoch unterhalb.

9. Hochfrequenzgenerator mit automatischer Leistungsregelung für die Hochfrequenzchirurgie nach Anspruch 1 bis 8, dadurch gekennzeichnet, daß
vor den Meßeingang (13) des phasengesteuerten Gleichrichters (11) ein Phasenschieber geschaltet ist, der bei der Frequenz der gleichzurichtenden Harmonischen eine solche Phasenverschiebung aufweist, daß die vom Lichtbogen erzeugte Harmonische nach dem Phasenschieber die vom phasengesteuerten Gleichrichter (11) geforderte Phasenlage, bezogen auf die vom Oszillator abgeleitete Steuerspannung am Steuereingang (13) des phasengesteuerten Gleichrichters (11), aufweist.

10. Hochfrequenzgenerator mit automatischer Leistungsregelung für die Hochfrequenzchirurgie nach Anspruch 1 bis 8, dadurch gekennzeichnet, daß
vor den Steuereingang (15) des phasengesteuerten Gleichrichters (11) ein Phasenschieber (18) geschaltet ist, der bei der Frequenz der gleichzurichtenden Harmonischen eine solche Phasenverschiebung aufweist, daß die vom Oszillator (1) abgeleitete Steuerspannung nach dem Phasenschieber (18) die vom phasengesteuerten Gleichrichter (11) geforderte Phasenlage, bezogen auf die vom Lichtbogen erzeugte Harmonische am Meßeingang (13) des phasengesteuerten Gleichrichters (11), aufweist.

11. Hochfrequenzgenerator mit automatischer Leistungsregelung für die Hochfrequenzchirurgie nach Anspruch 1 bis 8, dadurch gekennzeichnet, daß
als phasengesteuerter Gleichrichter (11) ein Quadraturdemodulator verwendet wird, dessen Ausgangssignal bei beliebiger Phasenlage zwischen dem Steuersignal (15) und dem Meßsignal (13) der Amplitude der gleichzurichtenden Harmonischen entspricht.

12. Hochfrequenzgenerator mit automatischer Leistungsregelung für die Hochfrequenzchirurgie nach Anspruch 1 bis 11, dadurch gekennzeichnet, daß
zwischen die Phasenmeßeinrichtung (4) und den ersten Regelverstärker (8) ein Sample-and-Hold-Verstärker geschaltet ist, der das von der Phasenmeßeinrichtung abgegebene Signal nur dann an den ersten Regelverstärker weitergibt, wenn der Hochfrequenzgenerator aktiviert ist und der dann in den Hold-Status gesteuert ist, wenn der Hochfrequenzgenerator nicht aktiviert ist, wobei er dann das letzte während einer Aktivierungsphase des Hochfrequenzgenerators von der Phasenmeßeinrichtung abgegebene Signal aufrecht erhält.

13. Hochfrequenzgenerator mit automatischer Leistungsregelung für die Hochfrequenzchirurgie nach Anspruch 1 bis 11, dadurch gekennzeichnet, daß
zwischen die Phasenmeßeinrichtung (4) und den ersten Regelverstärker (8) ein Sample-and-Hold-Verstärker geschaltet ist, der das von der Phasenmeßeinrichtung abgegebene Signal nur dann an den ersten Regelverstärker weitergibt, wenn der Hochfrequenzgenerator im Schneidmodus aktiviert ist und der dann in den Hold-Status gesteuert ist, wenn der Hochfrequenzgenerator im Koagulationsmodus oder gar nicht aktiviert ist, wobei er dann das letzte während einer Aktivierungsphase des Hochfrequenzgenerators im Schneidmodus von der Phasenmeßeinrichtung abgegebene Signal aufrecht erhält.

14. Hochfrequenzgenerator mit automatischer Leistungsregelung für die Hochfrequenzchirurgie nach Anspruch 1 bis 13, dadurch gekennzeichnet, daß
die gleichzurichtende harmonische Frequenz die dreifache Generatorfrequenz ist.

**Claims**

1. High frequency generator with automatic power control for high frequency electrosurgery, comprising an oscillator for generating the generator frequency, a modulator for controlling the output amplitude, a power amplifier for generating the necessary high frequency power, an output filter for suppressing frequencies other than the generator frequency of the high frequency generator and a harmonic measuring device for measuring frequencies harmonic to the generator frequency in the output circuit of the high frequency generator and produced by the arc existing between the surgical probe and the tissue to be cut during the cutting process, characterized in that
   a) the frequency of the oscillator (1) is adjustable with electronic means,
   b) at the output of the power amplifier (3) is provided a phase measuring device (4), which measures the phase shift between the output voltage and the output current of the power amplifier (3),
   c) the output signal of the phase measuring device (4) is so returned with the aid of a first variable-gain amplifier (8) to the frequency regulating input of the oscillator (1) that the phase shift between the output voltage and the output current of the power amplifier (3) corresponds at the frequency which is set to the ideal value for the power amplifier (3) and which is generally approximately 0° and
   d) the harmonic measuring device (10) contains a phase-controlled rectifier (11), whose control signal is derived from the instantaneous frequency of the oscillator (1) and which only rectifies the harmonic frequency contained in the frequency mixture at the output of the high frequency generator and the output signal of the harmonic measuring device (10) is supplied to the modulator (2) across a second variable-gain amplifier (20) as the control signal for controlling the output power of the high frequency generator.

2. High frequency generator with automatic power control for high frequency electrosurgery according to claim 1, characterized in that the output filter (5) is a band-pass filter, whose critical frequencies are so dimensioned that all generator frequencies occurring in operation are within these critical frequencies, but the harmonic frequency to be rectified falls outside the same.

3. High frequency generator with automatic power control for high frequency electrosurgery according to claim 1, characterized in that the output filter (5) is a low-pass filter, whose critical frequency is so dimensioned that all the generator frequencies occurring in operation are below this critical frequency, but the harmonic frequency to be rectified is above it.

4. High frequency generator with automatic power control for high frequency electrosurgery according to claims 1 to 3, characterized in that the output impedance of the output filter (5) at the harmonic frequency to be rectified is high and that the signal supplied to the harmonic measuring device (10) is derived from the output voltage of the high frequency generator.

5. High frequency generator with automatic power control for high frequency electrosurgery according to claims 1 to 3, characterized in that the output impedance of the output filter (5) at the harmonic frequency to be rectified is low and the signal supplied to the harmonic measuring device (10) is derived from the output current of the high frequency generator.

6. High frequency generator with automatic power control for high frequency electrosurgery according to claims 1 to 5, characterized in that upstream of the harmonic measuring device (10) is connected a measuring filter (14) for suppressing the generator frequency and whose attenuation at all the generator frequencies which occur is so high that the linearity range of the phase-controlled rectifier (11) is not exceeded by the measuring signal.

7. High frequency generator with automatic power control for high frequency electrosurgery according to claim 6, characterized in that the measuring filter (14) is a band-pass filter, whose critical frequencies are dimensioned in such a way that the harmonic frequency to be rectified is within the said critical frequencies at all the generator frequencies which occur, but the generator frequency is outside said critical frequencies.

8. High frequency generator with automatic power control for high frequency electrosurgery according to claim 6, characterized in that the measuring filter (14) is a high-pass filter, whose critical frequency is dimensioned in such a way that the harmonic frequency to be rectified at all generator frequencies occurring in operation is above this critical frequency, but the generator frequency is below it.

9. High frequency generator with automatic power control for high frequency electrosurgery according to claims 1 to 8, characterized in that upstream of the measuring input (13) of the phase-controlled rectifier (11) is connected a phase shifter, which at the frequency of the harmonic to be rectified has such a phase shift that the harmonic produced by the arc has, following the phase shifter, the phase position required by the phase-controlled rectifier (11), based on the control voltage derived from the oscillator at the control input (13) of the phase-controlled rectifier (11).

10. High frequency generator with automatic power control for high frequency electrosurgery according to claims 1 to 8, characterized in that upstream of the control input (15) of the phase-controlled rectifier (11) is connected a phase shifter, which at the frequency of the harmonic to be rectified has a phase shift such that the control voltage derived from the oscillator (1), following the phase shifter (18), has the phase position required by the phase-controlled rectifier (11), based on the harmonic produced by the arc at the measuring input (13) of the phase-controlled rectifier (11).

11. High frequency generator with automatic power control for high frequency electrosurgery according to claims 1 to 8, characterized in that a quadrature demodulator is used as the phase-controlled rectifier (11) and its output signal at random phase positions between the control signal (15) and the measuring signal (13) corresponds to the amplitude of the harmonic to be rectified.

12. High frequency generator with automatic power control for high frequency electrosurgery according to claims 1 to 11, characterized in that a sample and hold amplifier is connected between the phase measuring device (4) and the first variable gain amplifier (8) and only passes the signal supplied by the phase measuring device to the first variable gain amplifier if the high frequency generator is activated and is then controlled in the hold status if the high frequency generator is not activated and then maintains the last signal supplied by the phase measuring device during an activation phase of the high frequency generator.

13. High frequency generator with automatic power control for high frequency electrosurgery according to claims 1 to 11, characterized in that between the phase measuring device (4) and the first variable-gain amplifier (8) is connected a sample and hold amplifier, which only transmits the signal supplied by the phase measuring device to the first variable-gain amplifier if the high frequency generator is activated in the cutting mode and which is then controlled in the hold status if the high frequency generator is in the coagulation mode or not activated and it then maintains the last signal supplied by the phase measuring device during an activation phase of the high frequency generator in the cutting mode.

14. High frequency generator with automatic power control for high frequency electrosurgery according to claims 1 to 13, characterized in that the harmonic frequency to be rectified is three times the generator frequency.

**Revendications**

1. Générateur haute fréquence à régulation automatique de la puissance, pour la chirurgie à haute fréquence, générateur comprenant un oscillateur pour générer la fréquence, un modulateur pour régler l'amplitude de sortie, un amplificateur de puissance pour créer la puissance haute fréquence, nécessaire, un filtre de sortie pour éliminer les fréquences autres que la fréquence du générateur haute fréquence et une installation de mesure des harmoniques pour mesurer les fréquences harmoniques correspondant à la fréquence du générateur dans le circuit de sortie du générateur, et qui sont créés par l'arc électrique se formant entre la sonde de chirurgie et le tissu à couper au cours d'une opération d'incision, générateur caractérisé en ce que:

a) la fréquence de l'oscillateur (1) se règle avec des moyens électroniques,

b) une installation de mesure de phase (4) est prévue à la sortie de l'amplificateur de puissance (3), installation qui mesure le déphasage entre la tension de sortie et le courant de sortie de l'amplificateur de puissance (3),

c) le signal de sortie de l'installation de mesure de phase (4) est appliqué en retour à l'entrée de réglage de fréquence de l'oscillateur (1) par l'intermédiaire d'un premier amplificateur de réglage (8) pour que le déphasage entre la tension de sortie et le courant de sortie de l'amplificateur de puissance (3) arrive en général sensiblement à zéro degré pour la fréquence qui s'établit à la valeur idéale de l'amplificateur de puissance (3) et,

d) l'installation de mesure des harmoniques (10) comporte un redresseur (11) commandé en phase dont le signal de sortie dérive de la fréquence instantanée de l'oscillateur (1) et qui ne redresse qu'une fréquence harmonique contenue dans le mélange des fréquences à la sortie du générateur hautes fréquences et le signal de sortie de l'installation de mesure des harmoniques (10) est fourni par le modulateur (2) par l'intermédiaire d'un second amplificateur de réglage (20) comme signal de référence pour le réglage de la puissance de sortie du générateur hautes fréquences.

2. Générateur haute fréquence à régulation automatique de la puissance pour la chirurgie à haute fréquence selon la revendication 1, caractérisé en ce que :

- le filtre de sortie (5) est un filtre à bande passante dont les fréquences limites sont choisies pour que toutes les fréquences du générateur qui se produisent pendant son fonctionnement se situent à l'intérieur de ces fréquences limites, mais que la fréquence harmonique, à redresser se situe à l'extérieur des fréquences limites.

3. Générateur haute fréquence à régulation automatique de la puissance pour la chirurgie à haute fréquence selon la revendication 1, caractérisé en ce que :

- le filtre de sortie (5) est un filtre passe bas dont la fréquence limite est dimensionnée pour que toutes les fréquences de générateur qui se produisent pendant le fonctionnement se situent en dessous de cette fréquence limite et que la fréquence harmonique à redresser se situe au-dessus de cette fréquence limite.

4. Générateur haute fréquence à régulation automatique de la puissance pour la chirurgie à haute fréquence selon les revendications 1 à 3, caractérisé en ce que :

- l'impédance de sortie du filtre de sortie (5) pour la fréquence harmonique à redresser est fortement ohmique et en ce que le signal appliqué à l'installation de mesure harmonique (10) dérive de la tension de sortie du générateur haute fréquence.

5. Générateur haute fréquence à régulation automatique de la puissance pour la chirurgie à haute fréquence selon les revendications 1 à 3, caractérisé en ce que l'impédance de sortie du filtre de sortie (5) est faiblement ohmique pour la fréquence harmonique à redresser et en ce que le signal appliqué à l'installation de mesure harmonique (10) provient du courant de sortie du générateur haute fréquence.

6. Générateur haute fréquence à régulation automatique de la puissance pour la chirurgie à haute fréquence selon les revendications 1 à 5, caractérisé par un filtre de mesure (14) en amont de l'installation de mesure harmonique (10) pour supprimer la fréquence du générateur et dont l'amortissement pour toutes les fréquences existantes du générateur est tellement élevé que la plage de linéarité du redresseur commandé en phase (11) n'est pas dépassée par le signal de mesure.

7. Générateur haute fréquence à régulation automatique de la puissance pour la chirurgie à haute fréquence selon la revendication 6, caractérisé en ce que :
   - le filtre de mesure (14) est un filtre à bande passante dont les fréquences limites sont dimensionnées pour que la fréquence harmonique à redresser se situe pour toutes les fréquences de générateur susceptibles de se produire au cours du fonctionnement, à l'intérieur de ces fréquences limites et que la fréquence du générateur se situe néanmoins à l'extérieur des fréquences limites.

8. Générateur haute fréquence à régulation automatique de la puissance pour la chirurgie à haute fréquence selon la revendication 6, caractérisé en ce que :
   - le filtre de mesure (14) est un filtre passe haut dont la fréquence limite est choisie pour que la fréquence harmonique à redresser soit toujours située au dessus de cette fréquence limite pour toutes les fréquences qui se produisent pendant le fonctionnement du générateur de fréquences, la fréquence du générateur se situant néanmoins en dessous de cette fréquence limite.

9. Générateur haute fréquence à régulation automatique de la puissance pour la chirurgie à haute fréquence selon les revendications 1 à 8, caractérisé en ce que :
   - en amont de l'entrée de mesure (13) du redresseur (11) commandé en phase, est prévu un déphaseur, qui à la fréquence des harmoniques à redresser, crée un déphasage tel que l'harmonique générée par l'arc électrique présente la phase demandée par le redresseur (11) commandé en phase selon le déphaseur, phase rapportée à celle de la tension de commande provenant de l'oscillateur et qui est appliquée à l'entrée de commande (13) du redresseur (11) commandé en phase.

10. Générateur haute fréquence à régulation automatique de puissance pour la chirurgie à haute fréquence selon les revendications 1 à 8, caractérisé en ce que :
    - un déphaseur (18) est prévu en amont de l'entrée de commande (15) du redresseur (11) commandé en phase, ce déphaseur assurant un déphasage à la fréquence des harmoniques à redresser, telle que la tension de commande fournie par l'oscillateur (1) après le déphaseur (18) possède la position de phase demandée par le redresseur (11) commandé en phase, position rapportée à l'harmonique générée par l'arc électrique et qui est appliquée à l'entrée de mesure (13) du redresseur (11) commandé en phase.

11. Générateur haute fréquence à régulation automatique de la puissance pour la chirurgie à haute fréquence selon les revendications 1 à 8, caractérisé en ce que comme redresseur commandé en phase (11), on utilise un démodulateur en quadrature dont le signal de sortie correspond à l'amplitude des harmoniques à redresser quelle que soit la position de phase entre le signal de commande (15) et le signal de mesure (13).

12. Générateur haute fréquence à régulation automatique de la puissance pour la chirurgie à haute fréquence selon les revendications 1 à 11, caractérisé en ce qu entre l'installation de mesure de phase (4) et le premier amplificateur de réglage (8), il est prévu un amplificateur d'échantillonnage et de maintien qui transmet le signal fourni par l'installation de mesure de phase au premier amplificateur de régulation, seulement si le générateur haute fréquence est activé et qu'il est commandé à l'état de maintien, lorsque le générateur haute fréquence n'est pas activé, et dans ce cas il maintient en l'état le dernier signal fourni par l'installation de mesure de phase au cours de la phase d'activation du générateur haute fréquence.

13. Générateur haute fréquence à régulation automatique de puissance pour la chirurgie à haute fréquence selon les revendications 1 à 11, caractérisé en ce que :
    - entre l'installation de mesure de phase (4) et le premier amplificateur de réglage (8), il est prévu un amplificateur d'échantillonnage et de maintien qui ne transmet le signal fourni par l'installation de mesure de phase au premier amplificateur de réglage que si le générateur haute fréquence est activé en mode de découpage et il est commandé en mode de maintien si le générateur de hautes fréquence est en mode de coagulation ou n'est pas activés, et il maintient alors à l'état le dernier signal émis pendant la phase d'activation du générateur haute fréquence en mode de découpage par l'installation de mesure de phase.

14. Générateur haute fréquence à régulation automatique de la puissance pour la chirurgie à haute fréquence selon les revendications 1 à 13, caractérisé en ce que la fréquence harmonique à redresser est la fréquence triple de celle du générateur.

Fig. 1

EP 0 237 795 B1

Fig. 2

Fig. 3

EP 0 237 795 B1

Fig. 4